# EUROPEAN PATENT SPECIFICATION

(11) **EP 3 167 838 B1**
(45) Date of publication and mention of the grant of the patent: **17.02.2021**
(21) Application number: 15003244.9
(22) Date of filing: 13.11.2015
(51) Int. Cl.: F16M 11/06, F16M 11/20, F16M 13/02, A61B 90/50

(54) **ROTABLE CONNECTION HAVING A BRAKE MECHANISM**
DREHBARE VERBINDUNG MIT EINEM BREMSUNGSMECHANISMUS
CONNEXION ROTATIVE COMPORTANT UN MÉCANISME DE FREINAGE

(43) Date of publication of application: 17.05.2017
(73) Proprietor: Ondal Medical Systems GmbH, 36088 Hünfeld (DE)
(72) Inventor: OGINSKI, Stefan, 36041 Fulda (DE); HÖSER, Markus, 36142 Tann (DE)
(74) Representative: Graf von Stosch, Andreas

(56) References cited:
- EP-A2- 1 520 548
- US-A- 4 693 240
- US-A1- 2001 030 683
- US-A1- 2005 177 156
- US-A1- 2013 247 919
- US-B1- 6 632 170
- US-B2- 7 871 047

## Description

### Technical Field

The present invention relates to a rotatable joint having a brake mechanism according to claim 1. More particularly, the present invention relates to a brake mechanism which is preferably designed for use in a swivel or pivot joint of a supporting arm, such as the type used for supporting technical and/or medical equipment, e.g. in industrial environments, in hospitals or other medical environments.

Further, the present invention relates to a supporting arm system for supporting technical equipment, especially medical equipment in a hospital or other medical environment.

The present invention especially relates to a rotatable joint with individual features of claim 1 and to a supporting arm system for supporting technical equipment, especially medical equipment in a hospital or other medical environment, with individual features of the respective independent claim.

Supporting arm systems for supporting technical equipment, especially medical equipment in a hospital or other medical environment, comprise on ore more arms which are pivotably attached to a first element, such as a supporting structure, ceiling mount or other. Especially in the case of overhead mounting of these systems, the arms may be subjected to gravitational acceleration, when the axis of rotation of the rotatable joints of such systems is not exactly perpendicular to the gravitational field, which may be the case due to manufacturing tolerances. Accordingly brake mechanisms are arranged at the rotatable joints to enhance friction and to avoid such unintended movement of the arms of the system.

### Background of the Invention

The provision of brake mechanisms in a rotatable connection or a pivot connection for defining the resistance to a rotational movement of a support arm or mounting arm or other structure is known in the art.

US 2001/0030683 A1 discloses a surgical light apparatus includes a hub assembly configured to be attached to a ceiling, and an arm coupled for pivotal movement about the hub assembly. The ease with which connection hub rotates about rotation axis can be set by the user or assembly technician through adjustment of brake assembly. Brake assembly includes a brake pad, metal cylindrical sleeve, and brake adjustment screw. A shaft of rotatable joint extends through a brake pad. Brake adjustment screw extends through counter bored hole in main hub section and is received in tapped hole. Tightening and loosening of adjustment screw causes the frictional force exerted by brake pad on shaft to be increased and decreased respectively.

A disadvantage of these break assembly is the enhanced wear of the brake pad at the point where the adjustment screw exerts a pressure on the brake pad. Further, when a new break assembly gets operational the breaking forces may be reduced until the parts have become "ground-in".

### Summary of the Invention

Accordingly, it is an object of the present invention to provide an improved break assembly and a rotatable joint including such an improved break assembly.

The present invention provides a rotatable joint that comprises a first joint member for connection to a first element; a second joint member connected with the first joint member and configured for rotation relative to the first joint member about only one axis; and a break mechanism for adjusting the ease of rotation of the second joint member relative to the first joint member, the brake mechanism comprising: a first brake member annularly extending around the first joint member and being torque proof therewith and having a braking surface, a second brake member disposed at the second joint member for rotation therewith and having a braking counter-surface to abut against the braking surface of the first brake member, wherein the braking surface of the first brake member is spherically shaped and in that the braking counter-surface of the second brake member is correspondingly spherically shaped.
Due to the inventive spherical geometry of the braking surface and the braking counter-surface, wear is dramatically reduced and also frictional forces of the break mechanism needn't be re-adjusted for long time intervals. Even already right from the beginning of operation of a new rotatable joint including such break mechanism, breaking forces may be adjusted once without the need for early readjustment.
Further, due to the spherical symmetry, assembly during manufacturing is simplified, as the brake counter-surfaces of the second brake members have no preferred orientation.

In a preferred embodiment of the invention, the braking surface of the first brake member has a convex spherical shape and the braking counter-surface of the second brake member has a corresponding concave spherical shape. In an alternative embodiment, the braking surface of the first brake member may have a concave spherical shape and the braking counter-surface of the second brake member may have a corresponding convex spherical shape. Accordingly, the parts may be easily produced on a lathe.

In a preferred embodiment of the invention, the second brake member has a carrier part on which a braking shoe is axially movably arranged and wherein the braking counter-surface is arranged on a surface of the braking shoe and wherein the braking shoe is axially tensioned by means of at least one spring member arranged between the carrier part and the braking shoe. The spring member or spring members allow for a constant braking torque even in case of wear.

In a further preferable embodiment of the invention, the second brake member is formed as a braking screw, which carries a thread on a radial surface that cooperates with a threaded hole in the second joint member. Due to the thread which may be a fine pitch thread a precise adjustment of the braking torque is possible.

In a further preferable embodiment of the invention, the first joint member is configured or formed as a shaft and the second joint member is configured or formed as a sleeve which extends around the first joint member. The shaft may have at least in part a circular-cylindrical shape, while the sleeve may have at least in part a tubular circular-cylindrical shape and a diameter that allows placement of the sleeve around the shaft.

In a further advantageous embodiment of the invention, the first break member is formed as a separate, substantially ring-shaped part that extends around the shaft/first joint member. Such a ring-type break member may easily be produced. Advantageously, the first break member has at least one cam member for engagement with a notch arranged on the outer periphery of the shaft / first joint member and which extends in an axial direction. With the cam being located in the notch, a torque proof connection of the first break member and the shaft / first joint member may be achieved.

In an alternative embodiment of the invention, the first break member is formed as an integral part of the shaft.

According to another favourable aspect of the invention, the braking surface of the first brake member may be formed of one of bronze, brass, copper, steel, iron, ceramics, fibre-resin compounds or a combination thereof, generating a maximum wear resistance and optimal braking properties. Further, the braking counter-surface of the second brake member may be formed of one of bronze, brass, copper, steel, iron, ceramics, fibre-resin compounds or a combination thereof, again generating a maximum wear resistance and optimal braking properties.

In a further preferred embodiment of the invention, there are two or more second brake members provided at the second joint member. These two or more second brake members may cooperate with a single first brake member arranged at the shaft / first joint member or with more than one first brake member. With more than one second break member in place, braking torque may be enhanced and based on an evenly geometrical distribution of the second braking members around the outer diameter of the shaft, wear may further be reduced.

In a further preferred embodiment of the invention, the radius of the spherical braking surfaces is in the range of 8 mm to 150 mm, allowing for an optimized maximum braking torque / wear ratio.

The present invention provides a supporting arm system for supporting technical equipment, especially medical equipment in a hospital or other medical environment, having at least one supporting arm wherein the supporting arm comprises a rotatable joint according to any one of the preceding embodiments. The supporting arm system including the rotatable joint realizes all the advantages, as described above.

### Brief Description of the Drawings

The above and further features and advantages of the invention will become more readily apparent from the following detailed description of preferred embodiments of the invention with reference to the accompanying drawings, in which like reference characters identify like features, and in which:
Fig. 1 is a perspective side view of a rotatable joint in a supporting arm system with two supporting arms according to an embodiment of the invention;
Fig. 2 is a partial cut view along the line II - II in Fig. 1 showing the rotatable joint with the brake mechanism; and
Fig. 3 is an enlarged cut view of a first brake member of the brake mechanism of Fig. 2;
Fig. 4 is an enlarged partial cut view of a detail of Fig. 2 according to the marking IV in Fig. 2;
Fig. 5 is a partial cut view similar to that of Fig. 2 showing the rotatable joint with the brake mechanism in a further embodiment according to the invention;
Fig. 6 is an enlarged partial cut view of a detail of Fig. 2 according to the marking VI in Fig. 5;
Fig. 7 shows a cross section along the line VII - VII in Fig. 5 of the rotatable joint and the brake mechanism;
Fig. 8 is a perspective view of a second brake member of the break mechanism of Fig. 2.

### Detailed Description of the Preferred Embodiments

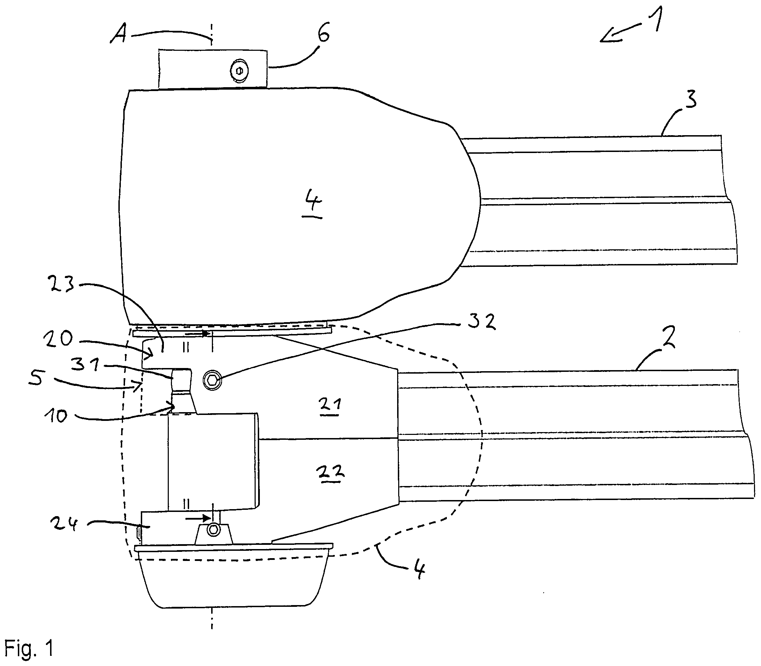

Referring to Fig. 1 of the drawings, a supporting arm system 1 according to a preferred embodiment of the invention are illustrated in a side view. The supporting arm system 1 has supporting arms 2, 3, each of the supporting arms 2, 3 is pivotably arranged on an axial structure 6 connected by means of rotatable joints 5. The axial structure may be part of or connected to one of a ceiling or overhead mount, a floor stand, a wall mount or a mobile stand etc., which is not shown in the drawings. Such supporting arm systems are especially used in the field of medical technology for the support of medical equipment or medical devices or for devices used in association with medical treatment rooms, such as medical lighting systems.

The supporting arms 2, 3 include support members (not shown in the figure) which are designed to support technical equipment, such as medical devices, medical service heads with service outlets, lighting, one or more computer display screen, and/or any of a variety of medical or industrial equipment for use in a corresponding environment (e.g. hospital, medical practice, factory etc.).

The rotatable joints 5 according to this embodiment of the invention are in the form of pivot joints or swivel joints for each of the supporting arms 2, 3. The rotatable joints 5 each comprise a first joint member 10 in the form of a substantially vertically extending cylindrical shaft or column, which is designed to be rigidly secured to a structure, such as a ceiling or overhead mount, a floor stand, a wall mount or a mobile stand etc. or directly to a ceiling of a building. The rotatable joints 5 may be covered by cover elements 4 which form a kind of protective casing. In Fig. 1 one of the cover elements 4 is made transparent for a better visibility of the underlying parts.
Furthermore, the rotatable joint 5 comprises a second joint member 20 which comprises a generally cylindrical sleeve part 23 which is mounted on an end of the first joint member 10 or shaft and extends around it. For a better visibility of the underlying parts, the sleeve part is made partially transparent in Fig. 1 as indicated by the dashed outer line of sleeve part 23. The second joint member 20 actually comprises a first part 21 and a second part 22, whereby the first part includes the sleeve part 23 while the second part includes the sleeve part 24. More particularly, the second joint member 20 is connected to the first joint member 10 for rotation relative to the first joint member 10 about a central axis A of the shaft / first joint member 10. That is, as can be seen in Fig. 1, the sleeve part 23 is mounted on upper and lower bearings 14, 15 (e.g. roller bearings) for rotary movement relative to the shaft / first joint member 10 about the central axis A.

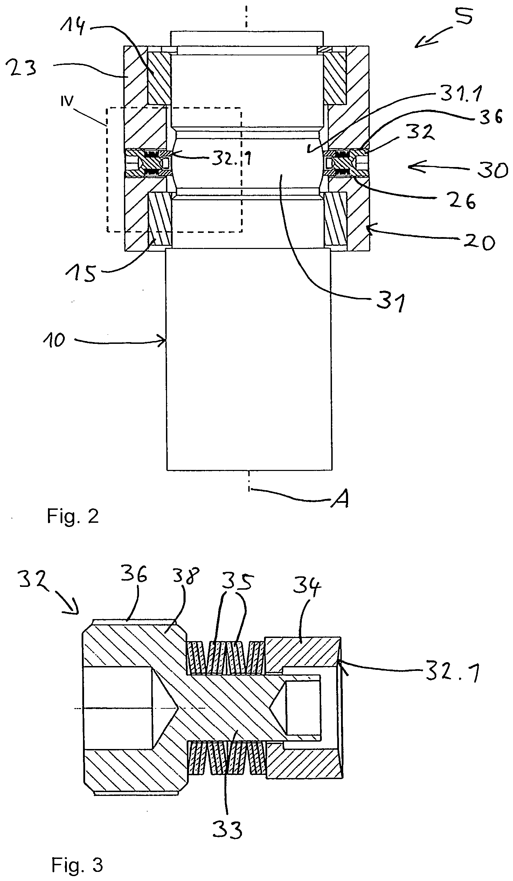

Details of the brake mechanism 30 will be more apparent from Figs. 2, 3 and 4 of the drawings, in which the rotatable joint 5 is illustrated in different views.

The brake mechanism 30 comprises a first brake member 31 which is generally ring-shaped and which extends around the outer periphery of the shaft / the first joint member 10. The first break member 31 is formed as a single piece with the shaft 10 and has a braking surface 31.1 with a convex spherical shape. The convex spherical shape has a radius R between 8 mm and 150 mm, preferentially between 20 and 50 mm. At least the braking surface 31.1 of the first brake member 31 may be formed of or comprise one of bronze, brass, copper, steel, iron, ceramics, fibre-resin compounds or a combination thereof. The brake mechanism 30 also comprises two second break members 32 in the form of two brake screws which are threaded in to a threaded hole 26 in the sleeve part 23 in the second joint member 20. The second break members 32 therefore carries a thread 36 on the radial outer surface of their heads 38. The second break members 32 has a concave spherically shaped braking counter-surface 32.1 which is located at the free end of a braking shoe 34 that is movably guided in a direction along a longitudinal axis of the second brake member 32 on a carrier part 33 of the second break members 32. At least the braking counter-surfaces 32.1 of the second brake members 32 may be formed of or comprise one of bronze, brass, copper, steel, iron, ceramics, fibre-resin compounds or a combination thereof. Radially outwardly of the carrier part 33 spring members 35 are arranged which contact on the one axial side the head 38 and on the other axial side the braking shoe 34. Spring m embers 35 in this embodiment are formed as disc springs but spring members may also be formed as other type of springs such as spiral springs etc. The second break members 32 are located on two opposite sides of the shaft 10 and are in respect to their longitudinal axis coaxially with each other but perpendicular to the central axis A, as can be seen in Fig. 2.

Frictional forces or respectively braking torque of the brake mechanism 30 may be adjusted by the position of the second brake members 32 in the threaded holes 26 in the second joint member 20. If the second brake members 32 are moved/screwed further inside the threaded hole towards the shaft 10, then the breaking torque is enhanced, while it is reduced in the other direction of movement / screwing of the second brake members 32. Consequently, braking torque effecting the supporting arms may be adjusted by adjusting the brake mechanism 30 as described above.

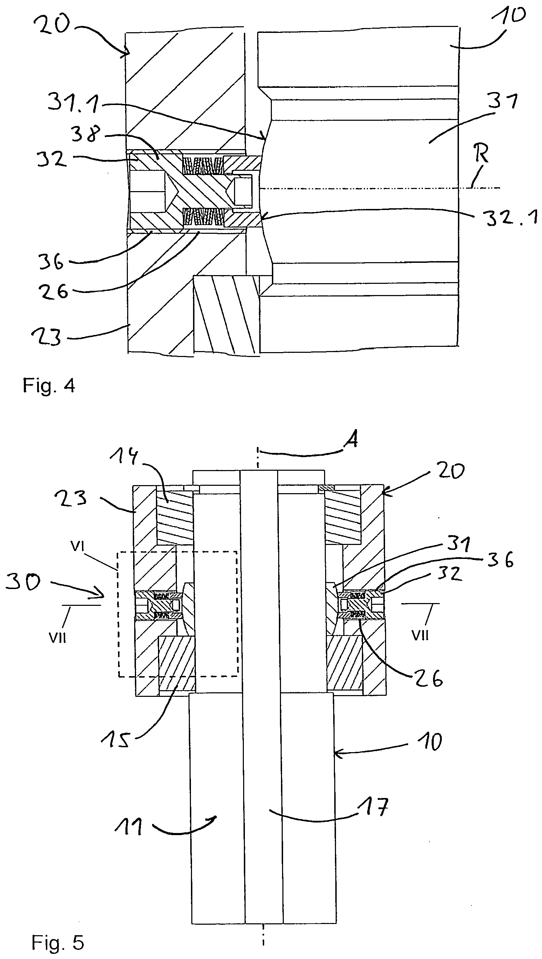

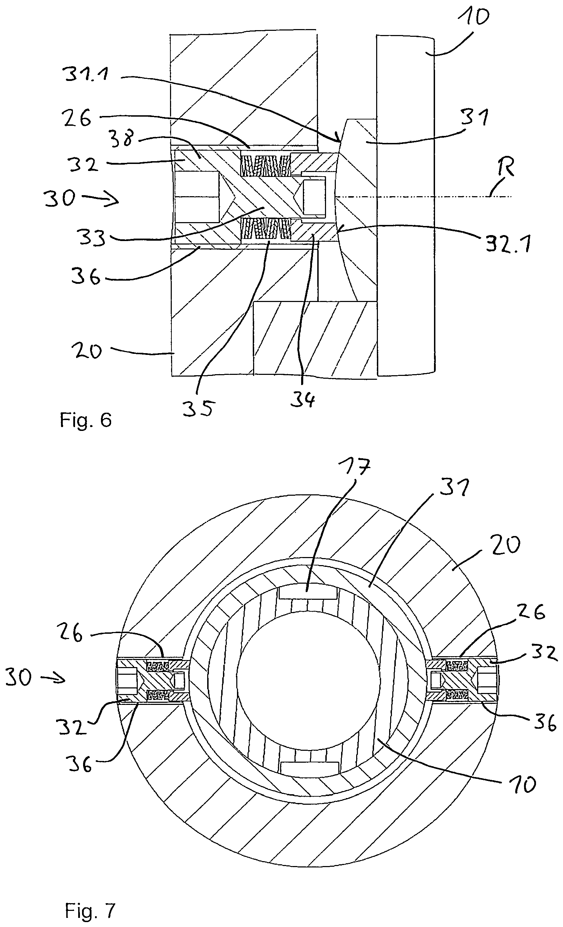

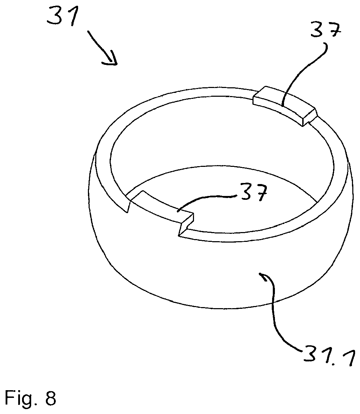

In Figs. 5 to 8 another favourable embodiment of the invention is illustrated. For reference numerals and functions not described in the following text, reference is made to the description of Figs. 1 to 4 which is herewith incorporated by reference.

This embodiment differs from the embodiment described with respect to figures 1 to 4 in that the first brake member 31 is formed as a separate, substantially ring-shaped part that extends around the shaft (10) and which has two cam members 37 located at an axial edge of the first brake member 31 on two opposite sides. The cam members 37 project inwardly from the ring-shaped first brake member 31 and comb respective notches 17 in the shaft / first joint member 10. The notches 17 are arranged on the outer periphery 11 of the shaft 10 and extend in an axial direction along central axis A. While a certain axial play is available between the shaft 10 and the ring-shaped first brake member 31, any pivoting or rotation of the first brake member 31 on the shaft or first joint member 10 is impeded by the cam members 37 in the notches 17.

It will be appreciated that the above description of the preferred embodiments of the invention with reference to the drawings has been made by way of example only. Thus, a person skilled in the art will appreciate that various changes, modifications and/or additions may be made to the parts particularly described and illustrated as long as they do not depart from the scope of the invention as defined in the claims. For example, although the shaft or column 10 / the first joint member 10 and the surrounding sleeve part 23 of the second joint member 20 have been shown in the drawings in a substantially vertical orientation, it will be apparent to a skilled person that the principles of the present invention may be applied with appropriate adaptation to other spatial orientations of the rotatable joint 5.

### REFERENCE LIST

- 1: supporting arm system
- 2: supporting arm
- 3: supporting arm
- 4: cover elements (for rotatable joints)
- 5: rotatable joints
- 6: axial structure

- 10: first joint member / shaft
- 11: outer periphery

- 14: upper bearings
- 15: lower bearings

- 17: notch

- 20: second joint member
- 21: first part
- 22: second part
- 23: sleeve part
- 24: sleeve part

- 26: threaded hole

- 30: brake mechanism
- 31: first brake member
- 31.1: braking surface
- 32: second brake member
- 32.1: braking counter-surface
- 33: carrier part
- 34: braking shoe
- 35: spring members
- 36: thread
- 37: cam member
- 38: head (of second brake member)

- A: axis
- R: radius

## Claims

1. A rotatable joint (5) for a supporting arm for supporting technical and/or medical equipment comprising:
a first joint member (10) for connection to a first element;
a second joint member (20) connected with the first joint member (10) and configured for rotation relative to the first joint member (10) about only one axis (A); and
a brake mechanism (30) for adjusting the ease of rotation of the second joint member (20) relative to the first joint member (10), the brake mechanism (30) comprising:
a first brake member (31) annularly extending around the first joint member (10) and being torque proof therewith and having a braking surface (31.1), and
a second brake member (32) disposed at the second joint member (20) for rotation therewith and having a braking counter-surface (32.1) to abut against the braking surface (31.1) of the first brake member (31),
wherein the braking surface (31.1) of the first brake member (31) is spherically shaped and in that the braking counter-surface (32.1) of the second brake member (32) has a corresponding spherical shape.

2. A rotatable joint (5) according to claim 1, **characterized in that** the braking surface (31.1) of the first brake member (31) has a convex spherical shape and the braking counter-surface (32.1) of the second brake member (32) has a corresponding concave spherical shape.

3. A rotatable joint (5) according to claim 2, **characterized in that** the second brake member (32) has a carrier part (33) on which a braking shoe (34) is axially movably arranged and wherein the braking counter-surface (32.1) is arranged on a surface of the braking shoe (34) and wherein the braking shoe (34) is axially tensioned by means of a spring member (35) arranged between the carrier part (33) and the braking shoe (34).

4. A rotatable joint (5) according to one of claims 1 to 3, **characterized in that** the second brake member (32) is formed as a braking screw, which carries a thread (36) on a radial surface that cooperates with a threaded passageway (26) in the second joint member (20).

5. A rotatable joint (5) according to any one of the preceding claims, **characterized in that** the first joint member (10) is formed as a shaft and the second joint member (20) is formed as a sleeve which extends around the first joint member (10).

6. A rotatable joint (5) according to any one of the preceding claims, **characterized in that** the first brake member (31) is formed as a separate, substantially ring-shaped part that extends around the shaft (10).

7. A rotatable joint (5) according to claim 6, **characterized in that** the first brake member (31) has at least one cam member (37) for engagement with a notch (17) arranged on the outer periphery (11) of the shaft (10) and which extends in an axial direction along axis (A).

8. A rotatable joint (5) according to any of claims 1 to 6, **characterized in that** the first brake member (31) is formed as an integral part of the shaft (10).

9. A rotatable joint (5) according to any one of the preceding claims, **characterized in that** the braking surface (31.1) of the first brake member (31) is formed of one of bronze, brass, copper, steel, iron, ceramics, fibre-resin compounds or a combination thereof.

10. A rotatable joint (5) according to any one of the preceding claims, **characterized in that** two or more second brake members (32) are provided at the second joint member (20).

11. A rotatable joint (5) according to any one of the preceding claims, **characterized in that** the braking counter-surface (32.1) of the second brake member (32) is formed of one of bronze, brass, copper, steel, iron, ceramics, fibre-resin compounds or a combination thereof.

12. A rotatable joint (5) according to any one of the preceding claims, according to any one of the preceding claims, **characterized in that** the radius (R) of the spherical braking surfaces (31, 32) is in the range of 8 mm to 150 mm.

13. A supporting arm system (1) for supporting technical equipment, especially medical equipment in a hospital or other medical environment, having at least one supporting arm (2, 3), wherein the supporting arm (2, 3) comprises a rotatable joint (5) according to any one of the preceding claims.

## Patentansprüche

1. Ein drehbares Gelenk (5) für einen Tragarm zum Tragen von technischen und/oder medizinischen Geräten, umfassend:
ein erstes Gelenkelement (10) zur Verbindung mit einem ersten Element;
ein zweites Gelenkelement (20), das mit dem ersten Gelenkelement (10) verbunden und zur Drehung relativ zu dem ersten Gelenkelement (10) um nur eine Achse (A) eingerichtet ist; und
einen Bremsmechanismus (30) zum Einstellen der Drehbarkeit des zweiten Gelenkelements (20) relativ zu dem ersten Gelenkelement (10), wobei der Bremsmechanismus (30) umfasst:
ein erstes Bremselement (31), das sich ringförmig um das erste Gelenkelement (10) herum erstreckt und mit diesem drehmomentfest ist und eine Bremsfläche (31.1) aufweist, und
ein zweites Bremselement (32), das an dem zweiten Gelenkelement (20) zur Drehung mit diesem angeordnet ist und eine Bremsgegenfläche (32.1) aufweist, um gegen die Bremsfläche (31.1) des ersten Bremselements (31) zu stoßen,
wobei die Bremsfläche (31.1) des ersten Bremselements (31) sphärisch geformt ist und dass die Bremsgegenfläche (32.1) des zweiten Bremselements (32) eine entsprechende sphärische Form aufweist.

2. Drehbares Gelenk (5) nach Anspruch 1, **dadurch gekennzeichnet, daß** die Bremsfläche (31.1) des ersten Bremselements (31) eine konvexe sphärische Form und die Bremsgegenfläche (32.1) des zweiten Bremselements (32) eine entsprechende konkave sphärische Form aufweist.

3. Drehbares Gelenk (5) nach Anspruch 2, **dadurch gekennzeichnet, dass** das zweite Bremselement (32) ein Trägerteil (33) aufweist, auf dem ein Bremsschuh (34) axial beweglich angeordnet ist, und wobei die Bremsgegenfläche (32.1) auf einer Oberfläche des Bremsschuhs (34) angeordnet ist und wobei der Bremsschuh (34) mittels eines Federelements (35), das zwischen dem Trägerteil (33) und dem Bremsschuh (34) angeordnet ist, axial vorgespannt ist.

4. Drehbares Gelenk (5) nach einem der Ansprüche 1 bis 3, **dadurch gekennzeichnet, dass** das zweite Bremselement (32) als Bremsschraube ausgebildet ist, die auf einer radialen Fläche ein Gewinde (36) trägt, das mit einem Gewindedurchgang (26) im zweiten Gelenkelement (20) zusammenwirkt.

5. Drehbares Gelenk (5) nach einem der vorstehenden Ansprüche, **dadurch gekennzeichnet, dass** das erste Gelenkelement (10) als eine Welle ausgebildet ist und das zweite Gelenkelement (20) als eine Hülse ausgebildet ist, die sich um das erste Gelenkelement (10) herum erstreckt.

6. Drehbares Gelenk (5) nach einem der vorstehenden Ansprüche, **dadurch gekennzeichnet, daß** das erste Bremselement (31) als ein separates, im wesentlichen ringförmiges Teil ausgebildet ist, das sich um die Welle (10) herum erstreckt.

7. Drehbares Gelenk (5) nach Anspruch 6, **dadurch gekennzeichnet, dass** das erste Bremselement (31) mindestens ein Nockenelement (37) zum Eingriff mit einer Einkerbung (17) aufweist, die am Außenumfang (11) der Welle (10) angeordnet ist und die sich in axialer Richtung entlang der Achse (A) erstreckt.

8. Drehbares Gelenk (5) nach einem der Ansprüche 1 bis 6, **dadurch gekennzeichnet, dass** das erste Bremselement (31) als ein integraler Teil der Welle (10) ausgebildet ist.

9. Drehbares Gelenk (5) nach einem der vorstehenden Ansprüche, **dadurch gekennzeichnet, daß** die Bremsfläche (31.1) des ersten Bremselements (31) aus Bronze, Messing, Kupfer, Stahl, Eisen, Keramik, Faser-Harz-Verbindungen oder einer Kombination davon gebildet ist.

10. Drehbares Gelenk (5) nach einem der vorstehenden Ansprüche, **dadurch gekennzeichnet, daß** zwei oder mehr zweite Bremselemente (32) an dem zweiten Gelenkelement (20) vorgesehen sind.

11. Drehbares Gelenk (5) nach einem der vorstehenden Ansprüche, **dadurch gekennzeichnet, daß** die Bremsgegenfläche (32.1) des zweiten Bremselements (32) aus Bronze, Messing, Kupfer, Stahl, Eisen, Keramik, Faser-Harz-Verbindungen oder einer Kombination davon gebildet ist.

12. Drehbares Gelenk (5) nach einem der vorstehenden Ansprüche, nach einem der vorstehenden Ansprüche, **dadurch gekennzeichnet, daß** der Radius (R) der sphärischen Bremsflächen (31, 32) im Bereich von 8 mm bis 150 mm liegt.

13. Ein Tragarmsystem (1) zum Tragen von technischen Geräten, insbesondere medizinischen Geräten in einem Krankenhaus oder einer anderen medizinischen Umgebung, mit mindestens einem Tragarm (2, 3), wobei der Tragarm (2, 3) ein drehbares Gelenk (5) nach einem der vorhergehenden Ansprüche aufweist.

## Revendications

1. Articulation rotative (5) permettant de supporter un bras destiné à soutenir un équipement technique et/ou médical comprenant :
un premier élément d'articulation (10) à raccorder à un premier élément ;
un second élément d'articulation (20) raccordé au premier élément d'articulation (10) et configuré afin de tourner relativement au premier élément d'articulation (10) autour de seulement un axe (A) ; et
un mécanisme de frein (30) permettant d'ajuster la facilité de rotation du second élément d'articulation (20) relativement au premier élément d'articulation (10), le mécanisme de frein (30) comprenant :
un premier élément de frein (31) s'étendant de manière annulaire autour du premier élément d'articulation (10) et étant résistant à la torque avec celui-ci et présentant une surface de freinage (31.1), et
un second élément de frein (32) disposé au niveau d'un second élément d'articulation (20) afin de tourner avec lui et présentant une contre-surface de freinage (32.1) permettant de venir en butée contre la surface de freinage (31.1) du premier élément de frein (31),
dans laquelle la surface de freinage (31.1) du premier élément de frein (31) est de forme sphérique et la contre-surface de freinage (32.1) du second élément de frein (32) présente une forme sphérique correspondante.

2. Articulation rotative (5) selon la revendication 1, **caractérisée en ce que** la surface de freinage (31.1) du premier élément de frein (31) présente une forme sphérique convexe et la contre-surface de freinage (32.1) du second élément de frein (32) présente une forme sphérique concave correspondante.

3. Articulation rotative (5) selon la revendication 2, **caractérisée en ce que** le second élément de frein (32) présente une partie de support (33) sur laquelle une semelle de freinage (34) est disposée axialement de manière mobile et dans laquelle la contre-surface de freinage (32.1) est agencée sur une surface de la semelle de freinage (34) et dans laquelle la semelle de freinage (34) est axialement tendue au moyen d'un élément à ressort (35) agencé entre la partie de support (33) et la semelle de freinage (34).

4. Articulation rotative (5) selon l'une des revendications 1 à 3, **caractérisée en ce que** le second élément de frein (32) est formé comme une vis de freinage, qui supporte un filetage (36) sur une surface radiale qui coopère avec un passage fileté (26) dans le second élément d'articulation (20).

5. Articulation rotative (5) selon l'une quelconque des revendications précédentes, **caractérisée en ce que** le premier élément d'articulation (10) est formé comme un arbre et le second élément d'articulation (20) est formé comme un manchon qui s'étend autour du premier élément d'articulation (10).

6. Articulation rotative (5) selon l'une quelconque des revendications précédentes, **caractérisée en ce que** le premier élément de frein (31) est formé comme une partie de forme sensiblement annulaire séparée qui s'étend autour de l'arbre (10).

7. Articulation rotative (5) selon la revendication 6, **caractérisée en ce que** le premier élément de frein (31) présente au moins un élément de came (37) permettant de se mettre en prise avec une encoche (17) ménagée sur la périphérie extérieure (11) de l'arbre (10) et qui s'étend dans une direction axiale le long d'un axe (A).

8. Articulation rotative (5) selon l'une quelconque des revendications 1 à 6, **caractérisée en ce que** le premier élément de frein (31) est partie intégrante de l'arbre (10).

9. Articulation rotative (5) selon l'une quelconque des revendications précédentes, **caractérisée en ce que** la surface de freinage (31.1) du premier élément de frein (31) est formée d'un composé parmi le bronze, le laiton, le cuivre, l'acier, le fer, la céramique, des composés en fibre-résine ou une combinaison de ceux-ci.

10. Articulation rotative (5) selon l'une quelconque des revendications précédentes, **caractérisée en ce que** deux seconds éléments de frein ou plus (32) sont ménagés au niveau du second élément d'articulation (20).

11. Articulation rotative (5) selon l'une quelconque des revendications précédentes, **caractérisée en ce que** la contre-surface de freinage (32.1) du second élément de frein (32) est formée d'un composé parmi le bronze, le laiton, le cuivre, l'acier, le fer, la céramique, des composés en fibre-résine ou une combinaison de ceux-ci.

12. Articulation rotative (5) selon l'une quelconque des revendications précédentes, **caractérisée en ce que** le rayon (R) des surfaces de freinage sphériques (31, 32) est compris dans la plage de 8 mm à 150 mm.

13. Système de bras de support (1) permettant de soutenir un équipement technique, en particulier un équipement médical dans un hôpital ou un autre environnement médical, présentant au moins un bras de support (2, 3), dans lequel le bras de support (2, 3) comprend une articulation rotative (5) selon l'une quelconque des revendications précédentes.
